# EUROPEAN PATENT APPLICATION

(11) **EP 0 692 272 A1**
(43) Date of publication of application: **17.01.1996**
(21) Application number: 95304932.7
(22) Date of filing: 14.07.1995
(51) Int. Cl.: A61M 5/50

(54) **Syringes**

(30) Priority: 14.07.1994 AR 32880394; 11.07.1995 AR 33272295
(71) Applicant: Marano, Carlos Jose, Buenos Aires (AR)
(72) Inventor: Marano, Carlos Jose, Buenos Aires (AR)
(74) Representative: Geering, Keith Edwin

(57) **Abstract**

A hypodermic non-reusable syringe having a predetermined limited nimber of strokes and comprising a barrel which has a rear end opening and at the opposite end is closed by a bottom provided with an outlet which extends through an external tubular adapter in which a tubular needle is engageable as a channelling means, for liquid contained in or to be contained in the barrel; a piston slidable in and longitudinally of the barrel and connected to a controlling plunger the opposite end of which projects through the barrel rear end opening; the piston being connected to the plunger via an engaging and disengaging mechamism of the piston and plunger in their aspiration and injection action; and retaining means for the plunger being provided within the barrel of the syringe in a region where it does not interfere with the normal operation of the piston and plunger.

## Description

### BACKGROUND OF THE INVENTION

This invention relates to improvements in hypodermic non-reusable syringes with predetermined limitation of strokes, and to a method of quick preparation of said syringes. The object of this invention is to provide a new syringe that, due to the way it has been developed, cannot be reused.

It is not necessary to explain many details to understand the importance of such a syringe, which assures that it will be used only by a single person, without the possibilty of reuse; once the suction and the ejection of medicine have been effected, the syringe becomes useless, without voluntary intervention by the user and regardless the desire of any intended subsequent user. In this way, the transmission of diseases as serious as Aids, hepatitis and blood contagion between an infected user and a healthy patient is avoided. It is known that one of the greatest risks of contagion is by reuse of disposable hypodermic syringes and needles.

To avoid this, devices to allow the disposal of contaminated material, such as boxes and containers which allow the insertion of syringes and needles, but obstruct their outlet, have proliferated.

The fact is that if the disposal of the material depends on the user's desire, the patient will lack every possible protection.

Non-reusable syringes of different types are known, such as those which withdraw the needle within the cavity of the barrel, in such a way that said needle obstructs the operative function of the syringe and itself becomes useless.

Likewise, there exist non-reusable syringes in which the piston, after the first suction and compression, meets a blocking mechanism which prevents new withdrawal, the piston becoming useless against the bottom of the barrel, without any possibility of motion.

In spite of the operating advantages provided by said known syringes,practice demonstrated that they present various problems which go against their acceptance and use.

One of these problems is that arising from major structural complexity as, even in the case of syringes which are functionally efficient, these demand a complex process of construction - which apart from limiting large scale production considerably raise the costs of production whereas low cost production is required.

Another problem to be considered, pertaining also to the final costs of the product, is the factibility of the manufacturing process. There exist syringe structures that have a device limiting the number of strokes, the effectiveness of which can be demonstrated in a prototype, but the truth is that they greatly complicate the production processes and oblige creation and development of new machinery and construction methodology, requiring substantial investment, all with considerable increase of the operational and production costs and with uncertain result.

The syringe of this invention is a non-reusable type of syringe, this being so by means of the resource of separating the piston and its stem by means of a mechanical fuse through an engaging and disengaging mechanism of the piston with respect to the stem, operated by the normal movement of the piston and the stem in their suction and ejection action, and providing means of keeping the stem within the syringe barrel. Moreover, this mechanism limits the strokes and is composed of two pieces that, apart from being respectively attached to said piston and the mentioned stem, are linked by a guiding means which is joint to the stem and a guided means which is joint to said piston; the guiding means forms a passage which has a first retaining section and a second section for releasing said guided means components over a single complete stroke; the guided means includes two bolts placed in an end or rear end of the piston, and mounted on a system of reversible support means.

This new means of circumstantial link between the piston and the plunger is much simpler, easier to manufacture and safer than the already known ones, but with the particular feature of having solved the problem of insertion of the guided means in the guiding one, in such a way that it can be constructed manually or by means of simple mechanical devices quickly, with high productivity and at low cost.

Likewise, and with an equivalent level of construction simplicity, the invention anticipates the production of non-reusable syringes with the indicated characteristics, but with the capacity of being useful for more than one complete stroke (for example 2 or 3 strokes). This is necessary for the case when it is necessary to subsequently inject the same or different medicines, or mix them, in subsequent suctions and ejections.

This is the case of medicines that include the syringe for its specific application; being prepared for the exact number of comlete strokes that said mixture requires, after which the plunger will become separated from the piston. To such effect it shall include more than one guiding means (one per stroke), combining their respective slides. Through the additional improvements of this invention a non-reusable syringe has been achieved, without the voluntary intervention of the user, that allows the movement of the piston and the plunger without putting into motion the mechanism that produces the disposal of the syringe, presenting a proper handling of the syringe in the steps previous to the load of the liquid to be injected. The operation of the releasing mechanism is produced when the liquid is loaded through the needle put in the syringe and when the liquid is ejected through the same for its application to the patient.

Therefore, the acceptance that the new improved syringe will have when put into practice is to be evident, whatever the category and purpose may be; its characteristics suit it equally as a non-reusable syringe without any voluntary intervention of the user and of limited and predetermined strokes, for medical or surgical use, in human medicine and even veterinary medicine.

### II - DESCRIPTION OF THE DRAWINGS

The invention is illustrated in the many figures of the accompanying drawings, in which it is shown in preferred embodiments in order to be clearer and better understood, all by way of illustrative non-limitative example.

Figure 1 is a longitudinal sectional view of the barrel, showing the end of the plunger with the guiding means, before being inserted through the bolts that are separated, before being reversed according to the arrows, when inserting the piston and said end of the plunger through the rear opening of said barrel.

Figure 2 is another longitudinal sectional view of the barrel, corresponding to the forward end which continues with the adapter for attachment of the needle. In this figure, one can see the piston with its guiding means already connected to the end of the plunger through the guided means (bolt) that is closed according to the small arrows to establish a single guided bolt.

Figure 3 is a longitudinal sectional view of the syringe construction, and with the wings that close the opening of the rear end reversed, showing the guided position of the bolt within the clip, when the first suction stroke is made.

Figure 4 is a sectional view similar to figure 3, but showing the guided position of the bolt when the piston moves as the arrows indicate for the compression operation (that in to say, for injecting).

It is observed that, in these circumstances, the bolt gets derailed according to the small arrow, coming out from the guiding means.

Figure 5 is a sectional view similar to figure 3, but showing the position in which the bolt has been disengaged from the guiding means and leaves the piston free from the plunger and incapable of reuse.

Figure 6 is a sectional view of the plunger through plane VI-VI in figure 3, allowing a transverse view of said plunger, which extends through a passage formed for such purpose in the rear end entrance of the barrel.

Figure 7 is a view of the end of the plunger and its guiding means from plane VII-VII in figure 5, showing how said guiding means is intercalated among the legs formed by said end.

Figure 8 is a view of the piston base from plane VIII- VIII in figure 5 provided at the same time by each projecting body that matching the arms forms a support of the parts which compose the bolt.

Figure 9 is a view of the piston and its back zone, without one of the bodies which compose it, to show the way in which the arms supporting the portions of the bolt are connected in an articulate way with the base of the piston.

Figure 10 is a side view of the guiding means to show its configuration in (an open "9") and the disposition of the different parts which form it defining the different guide parts for the bolt, according to the position of the piston.

Figure 11 is a detailed perspective exploded view of parts of the device of the present invention including the end of the plunger with the guiding means, unlatching bolt of the guided means, and associated parts.

Figure 12 illustrates in detail the guided means in its embodiment for two strokes.

Figure 13 shows a longitudinal sectional view of a syringe barrel and fragmentary sectional views of the piston which include the guiding and the guided means of this invention.

Figure 14 is a sectional view of the piston along line II-II of Figure 13 and shows the guiding means and the guided means separately.

Figure 15 is a sectional view similar to figure 14, where the process of construction of the guiding means with respect to the guided means is shown.

Figure 16 is a view in lateral elevation of the guiding means in its position of assemblage with the guided means.

Figure 17 is a view similar to figure 16, where the portion of the guided and guiding means in the ejection movement is illustrated.

Figure 18 is a view similar to figure 16, where the position of the guided and guiding means is illustrated, showing the projection of the guided means in its retaining position in dotted lines and in half retaining position in a complete line.

Figure 19 is a view similar to figure 16, where the position of the guided and guiding means is illustrated, showing the projection of the guided means in a complete line in its releasing and disengaging position of both when the ejection stroke is made.

Figure 20 shows the disengaging position of the guiding and guided means and the retention effect of the plunger within the syringe barrel.

Figure 21 shows a view from above of the guided means.

Figure 22 shows a view from below of the guiding means.

Figure 23 shows a lateral view in elevation and a fragmentary sectional view of the quiding means.

Figure 24 is a perspective view of the guided and guiding means in their disengaging position.

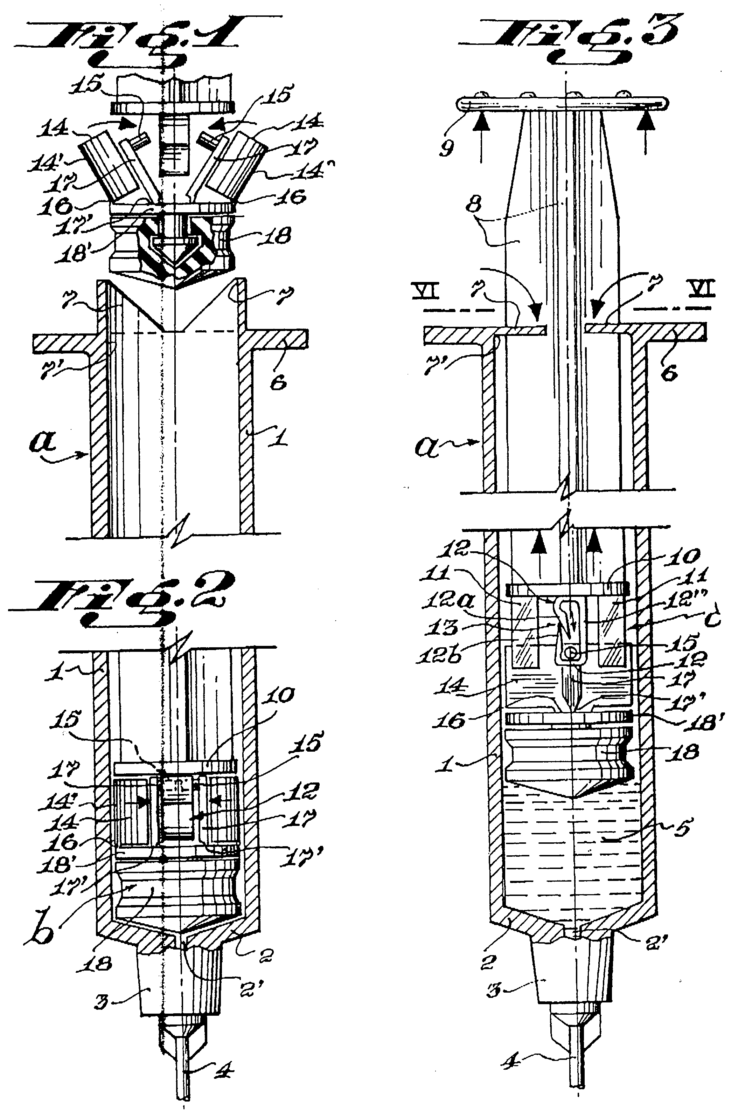

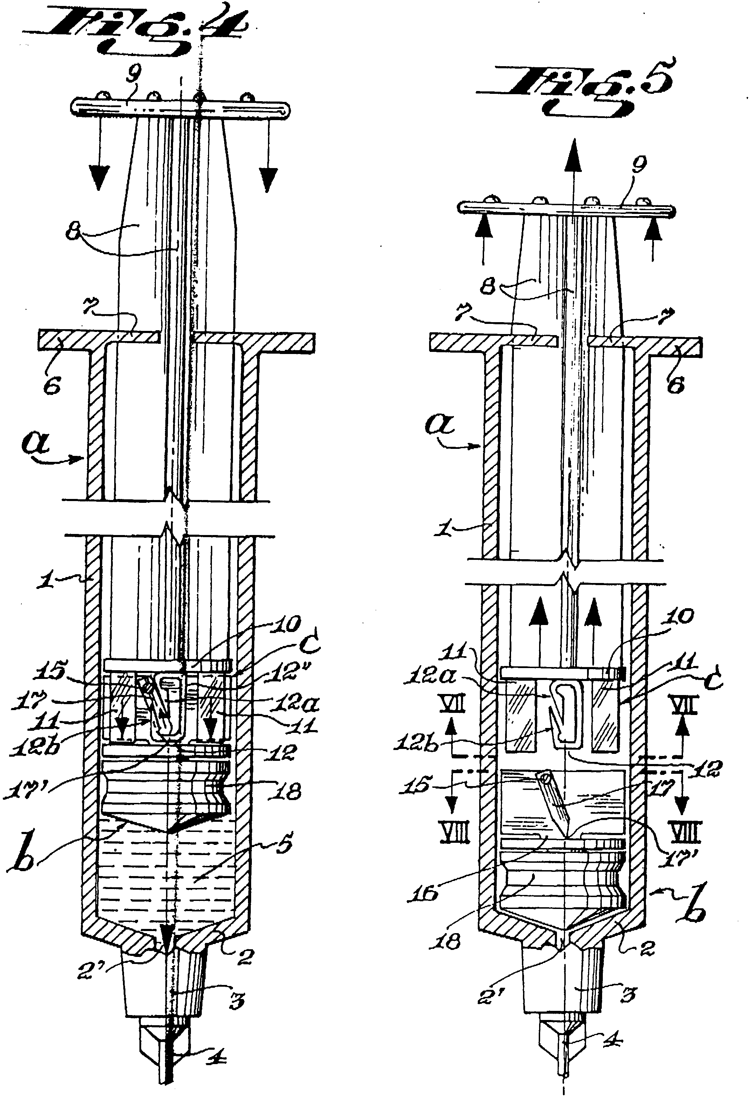

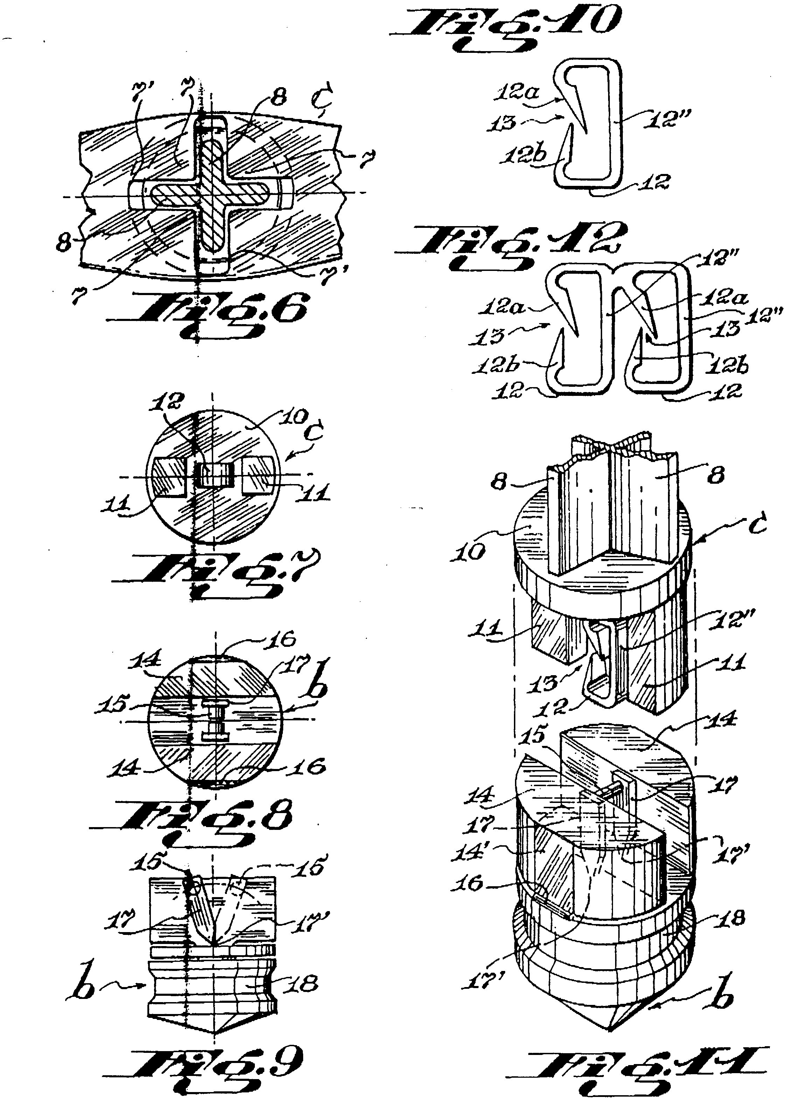

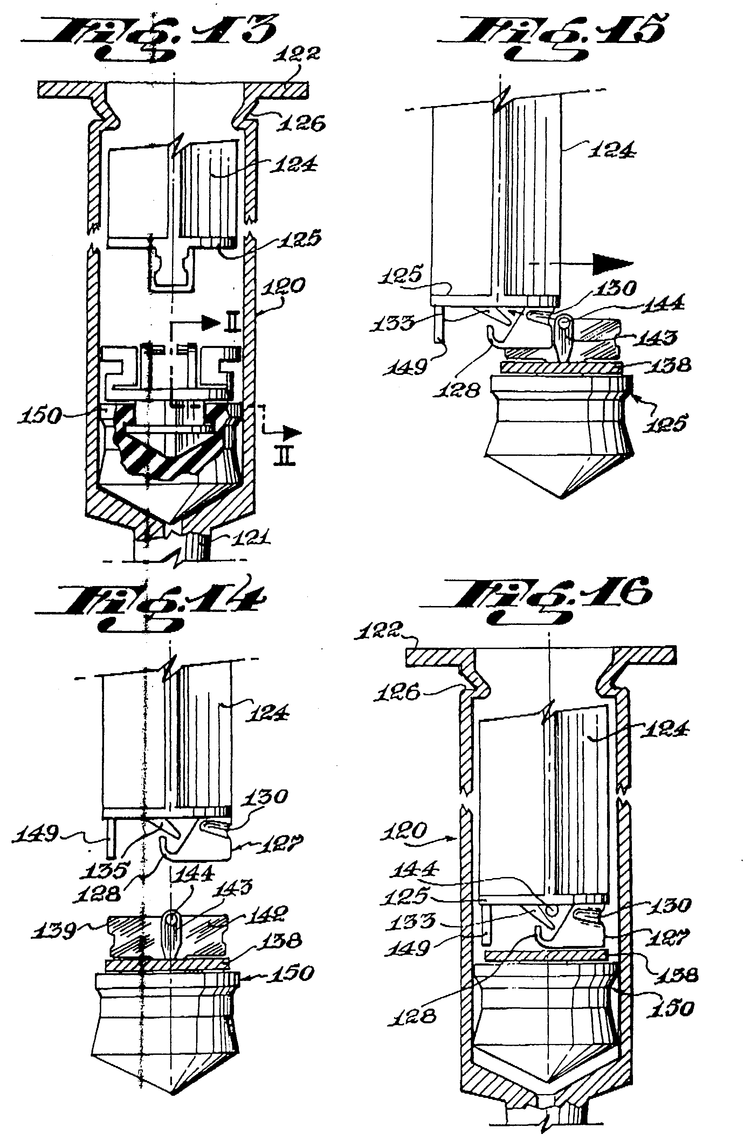

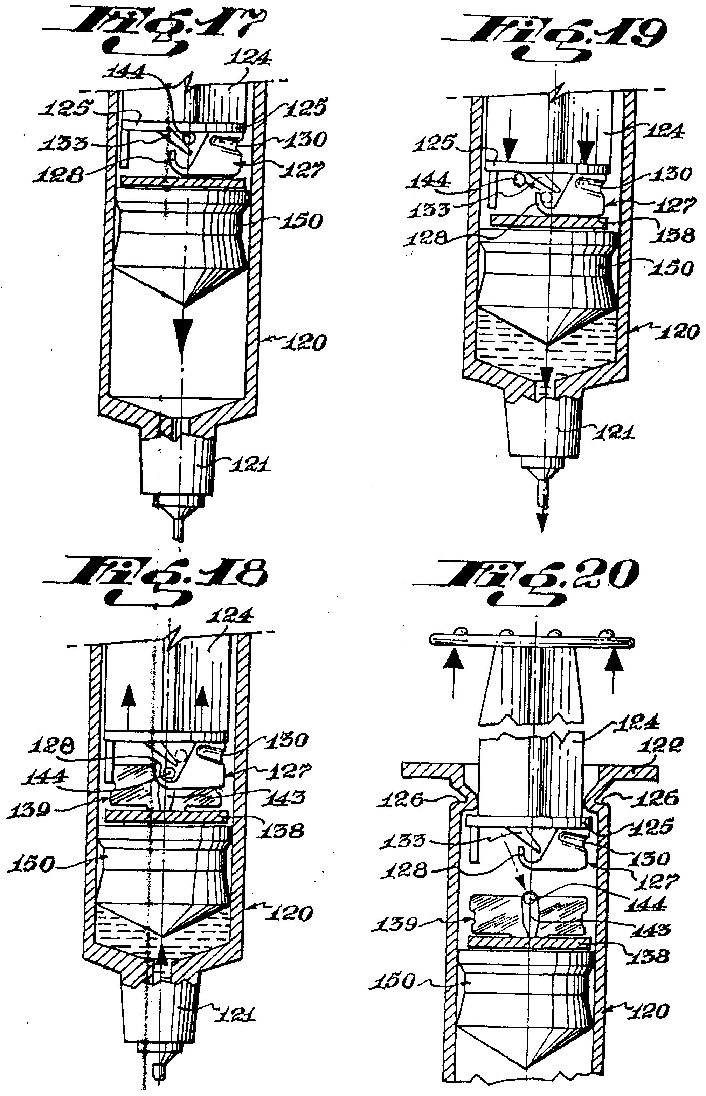

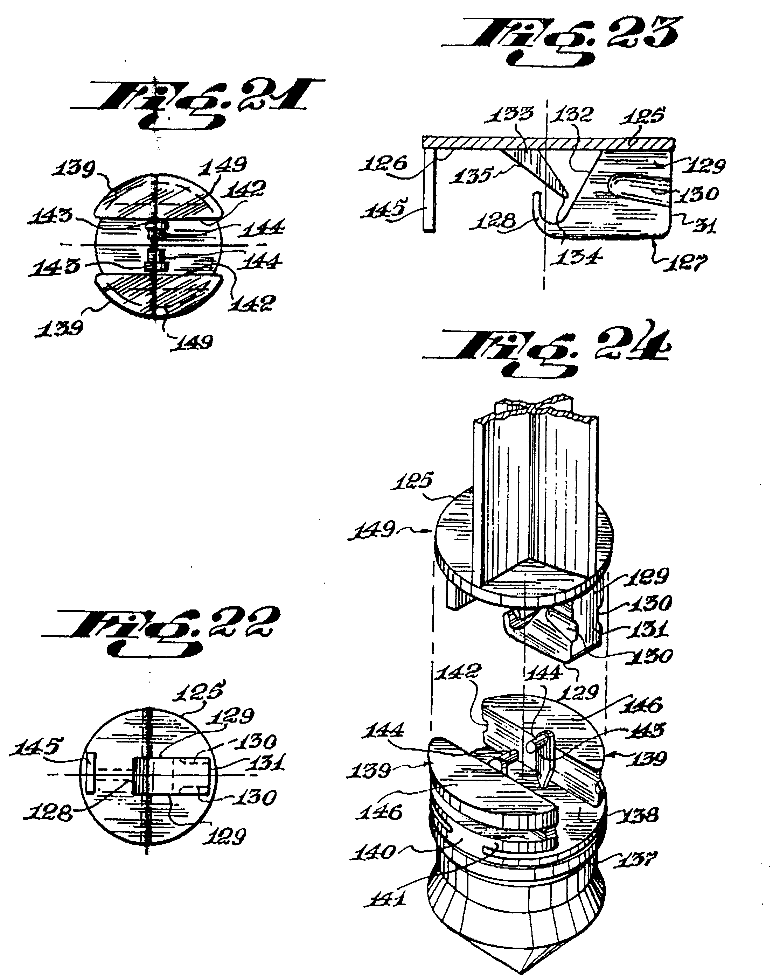

### III OBJECT OF THE INVENTION

Improvements in hypodermic non-reusable syringes without voluntary intervention of the user and with predetermined limitation of strokes, of the type that comprises an elongated barrel (a) which in one part comprises rear end opening (7') while in the opposite end it is closed with a bottom (2) provided with a perforation (2') extended through an external tubular adapter (3) in which a tubular needle (4) in engaged as a channelling means of the liquid contained in the barrel (a); said barrel (a) gives a sliding direction to a piston (18) connected with a controlling stem (8) the opposite end of which projects through the rear end opening (7'); characterized in that the piston (18) is connected to its stem (8) through an engaging and disengaging mechanism of the piston (18) with respect to the stem (8), operated by the normal movement of the piston (18) and the stem (8) for suction and ejection, retention means of the stem (8) being supplied within the barrel (a) of the syringe in an area where it does not interfere with the normal operation of the piston and the stem.

Another additional object of this invention is an engaging and disengaging mechanism which comprises a guiding means joint with said plunger and a guided means joint to said piston, said guiding means forming a passage which has a first retaining portion and a second releasing portion for said guided means.

Another additional object of this invention is the fact that said guided means is formed by a support disposed in the passage of the guiding means and connected to said piston in a laterally displaceable way with respect to the movement of the mentioned piston.

Another additional object of this invention is the fact that said support comprises two bolts, one opposite the other, placed in said passage of the guiding means and each connected by its external end to a respective leg elastically articulated to the upper base of the piston.

Moreover, another additional object of this invention is the fact that the piston has a circular base which, in its surface opposite to the fixing of the legs, has a retaining means of a cylindrical body constituting the piston itself which makes contact in an hermetically displacing way with the inner wall of the barrel.

Another additional object of this invention is improvement in hypodermic non-reusable syringes of the type that comprises an elongated barrel which in one end is open while in the opposite end it in closed with a bottom provided with a pipe through an external tubular adapter for the engagement of a needle, a piston placed in a sliding way in said control barrel by a plunger, the opposite end of which is projected further than the opening end of the barrel, the piston being connected to the plunger through an engaging and disengaging mechanism of the piston with respect to the plunger, with retaining means for the plunger within the syringe barrel in an area where it does not interfere with the normal operation of the piston and the stem, said engaging and disengaging mechanism comprising a guiding means joint to the stem and guided means joint to the piston, said guiding means having a passage which has a first retaining portion and a second releasing portion for said guided means and said guided means held by a projection in said passage of the guiding means, characterized in that in said passage are elastic retaining means of said projection, capable of allowing the movement of the piston according to an established condition and the release of mentioned projection according to another established condition, of retaining means for the displacement of said projection in the retaining and releasing portions of said passage of the guiding means.

### IV - DESCRIPTION

This invention refers to improvements in hypodermic non-reusable syringes without voluntary intervention of the user with predetermined limitation of strokes, as well as the quick construction method for said syringes.

Said improvements have been practised in a syringe comprising an elongated barrel (a) that, while in one end it has a rear end opening (7') in the opposite end it is closed with a bottom (2) provided with a prolonged perforation (2') through an external tubular adapter (3). Said adapter (3) engages a needle (4) which constitutes a channelling means of the liquid contained in barrel (a).

This barrel (a) gives sliding direction to a piston (18) which is connected to a controlling plunger (8). This last (8) has a body formed as radially expanded ribs that at its opposite end projects through the rear end opening (7') to finish in the final thrust end (9) of the plunger (8).

Furthermore, the piston (18) is connected to the plunger (8) through an engaging and disengaging mechanism limiting of strokes which is comprised by two sets of pieces: the guiding means (c) and the guided means (b).

The guiding means (c) is formed by the plunger (8), by the support base (10) which is at the inner end of said plunger (8), by the thrust legs (11) and by the guiding member (12). This last (12) is joint to the central area of said support base (10) while in both sides of the guiding member (12) and in a coplanar position with the same, the thrust legs (11) are placed.

In a more detailed way the guiding member (12) is an elongate piece of adequate width which has a longitudinal portion(12'') approximately directed parallel to the geometrical longitudinal axis of the barrel (a) and an upper elbow fixed to the lower surface of the support base (10) which finishes in a lateral portion (12a) directed against the support base (10) and slightly displaced towards the longitudinal portion (12''), and a lower elbow opposite to the upper elbow which finishes in a lower lateral portion (12b) opposite to the longitudinal portion (12'') and directed towards the support base (10), but which externally overlaps the end of the lateral portion (12a) of mentioned upper elbow, leaving an empty space between them. The thickness of mentioned upper (12a) and lower (12b) lateral portions is such as to allow elastic displacement of their ends, so that they then return to their initial position.

Said upper portion (12a), the upper elbow and the longitudinal portion (12'') form the retaining portion for the guided means (b) and the lower portion (12b) with the end directed towards the inside of the upper section (12a) forms the releasing portion for the guided means (b).

Regarding the guided means, it is a bolt (15) divided into two complementary sections projecting from each support leg (17). These finish in ends (17') which articulate in the base (18') of the piston (18). In this way, the bolt (15) is placed in the central area of said base (18'') and within a deep neck formed by two bodies (14) of circular external outline and internally flat which project from the base (18'). Besides, the mentioned bodies (14) are joined by means of articulations (16) with the mentioned base (18') of the piston (18).

In different embodiments, two or three combined guiding means (12) can define outlet (13) channels of the piston (15) corresponding to two or three complete strokes, respectively, as is shown in figure 12.

In the other end of the syringe, the rear and opening (7') of the barrel (a) has deforming wings (7) over said opening (7'), defining a polygonal passage coincident with the transverse section of the plunger (8), in such a way that said wings, so folded over the mentioned opening (7'), constitute retaining tops for the piston (18) in the assembled syringe.

In a different embodiment, the wings (7) can be replaced by deformations of the lateral wall (1) of the barrel (a) towards the inside of the same.

### OPERATION OF THE SET

The set works in the following way:
The folding wings (7) of the rear end (7') opening of the barrel being open, when the guided set (b) and the guiding set (c) are inserted, the bodies (14) of the piston (18) which are open, are displaced by the walls (1) of the barrel (a) in such a way that, in turn, they displace the support legs (17) of the bolt (15). Consequently, the complementary portions of said bolt (15) approach closing within the guiding member (12) of the guiding set (c).

As the set within the barrel (a) goes on moving, the two portions of the bolt (15) join and together with the union area covered by the guiding means (12) they position against the end of said member (12) next to the support base (10).

Once the piston (18) bumps into the bottom (2) of the barrel (a), the suction stroke begins, and the bolt (15), disposed in the member(12), moves against the end of said member (12) distant from the supporting base (10).

Once the suction in over, for compression operation (to inject) the plunger moves forward. With this movement, the bolt (15), which was against the end of the member (12) distant from the support base (10), moves along the longitudinal portion (12'') until the height of the lateral portion(12a), where the proximity of the last one with the longitudinal portion (12''), diverts a portion of the bolt (15) which goes into an outlet channel (13) limited by extreme teeth (12a) and (12b).

This construction determines that the movement of the bolt (15) shall load it to the outlet of the guiding means (12) and, therefore, to the disengagement between the plunger (8) and the piston (18).

### ASSEMBLY METHOD:

The method consists in that the piston (18) of the guided set (b) goes into the rear end opening (7') of the syringe barrel (a).

After that, the guiding means (12) is placed by leaning it on the central area of the base (18') of the piston (18) in such a way that the bolt portions (15) projecting from the support legs (17) face the slides formed by the longitudinal portion (12'') and the teeth (12a) and (12b) of said guiding means (12).

Then, from the plunger (8), the piston (18) is pushed, (18), inserting it in the barrel (a) and forcing with the lateral walls (1) of the latter (a) the rebuttal of the bodies (14) and the support legs (17) which are open, until they close making the insertion of the portions of the bolt(15) within the slides formed by the guiding means (12).

Finally, the folding wings (7) limiting the rear end opening (7') of the barrel (a) in connection with the radial ribs of the plunger (8) are folded, which, in some embodiments, can be by means of a heating method that fixes the fold of the mentioned folding wings (7).

A preferred embodiment of improvement in hypodermic non-reusable syringes, without the voluntary intervention of the user, and without limitation of strokes except for the charging and discharging of liquid under pressure, is the one referred to hereafter:

The syringe to which said improvements have been applied comprises a conventional barrel (120), which at one end has an adapter (121) to engage a needle (not illustrated) in a free removable way, also conventional. At the opposite open end of the mentioned barrel (120) is a conventional flange (122) through which the stem (124) of the piston (123) moves, the latter being provided with a disk (125) the diameter of which is slightly smaller than the inner diameter of the barrel (120) and limits the plunger (124) stroke when it bumps into at least two inward projections or deformations (126) in the wall of the barrel (120) adjacent to the flange (122) of the open end of mentioned barrel (120).

The piston (123) is composed of a guiding set (149) and a guided set (150) . The guiding set (149), as illustrated in figure 23, has a disk (125) from whose surface (126) a body (127) projects radially. Said body extends from the outline of the mentioned disk (125) up to a little bit more than the central axis of the barrel (120), and ends in a projection (128) that curves towards the disk (125) in a way parallel to the virtual axis of the mentioned barrel (120), remaining at an appropriate distance from the free surface (126) of the disk (125). The width of said body (127) is substantially smaller than the radius of said disk (125), and in each lateral wall (129) it is provided with a respective groove (130) which tapers from a wider end at outer wall (131) towards the inner wall (132), which inner wall presents an inclined plane ending in the mentioned projection (128).

From the free surface of the disk (125) a wing (133) projects (the width of the wing being the same as that of the body (127) ) and extends from the semicircumference opposite to the body (127), adjacent to the longitudinal axis of barrel (120) under the projection (128) and facing the inner wall (132) of the body (127), leaving a free space (134) between them. The external surface (135) of the wing (133) leaves a free space (136) between itself and the end of projection (128).

The guided set (150) is composed of a conventional cylindrical cap (137), which in its surface opposite to the adapter (121) has a disk (138) joint to the cap (137). The disk (138) in its free surface has two projecting bodies (139), the transverse sections of which are circular segments; they are joined to the mentioned disk (138) by an extension (140) of the contiguous outline of the curved surface (141) of mentioned projecting bodies (139). The separation between the flat surfaces (142) of the projecting bodies (139) is slightly bigger than the width of the body (127), and they are slightly higher than said body (127).

On the diameter of the disk (138) perpendicular to the flat surfaces (142), two facing supports (143) are disposed adjacent to the flat surfaces (142) and end in respective cylindrical projections (144) which are perpendicular to the supports (143), in longitudinal alignment, and leave a free space between themselves. The joint of the supports (143) to the free surface of the disk (138) is such that it allows radial movement of them parallel to the flat surfaces (142).

It is important to notice that the diameter of the cylindrical projections (144) in slightly bigger than the free spaces (134) and, (136) of the guiding set (149), so that the passage of the cylindrical projections (144) through the free spaces (134) and (136) should occur pursuant to certain conditions of pressure in the guided means (150) and in the guiding means (149), respectively.

The assembly process of the syringe consists firstly in engaging the guiding set (149) with the guided set (150), and then in the insertion of the piston (123) so formed, by pushing the plunger (124), up to the inside of the barrel (120). Once the disk (125) has been placed beyond the flange (122), by proper means, the deformation of the wall of the barrel (120) produces the obstructions (126), which impede the removal of the piston (123) and the stem (124) from the inside of the barrel (120). The engagement of the guiding set (149) with the guided set (150) is performed in the way illustrated in figure 15, where the cylindrical projections (144) face the grooves (130) and slide laterally along them towards the inner wall (132) of the body (127) together with the the other elements of the guided set (150) for elastically forcing the supports (143) in the last portion of the lateral walls (129) before reaching the assembly position, that in when the projections (144) are disposed in the space limited by the free surface of the disk (125) the inner wall (132) of the body (127) and the wing (133), as is illustrated in figure 16.

### OPERATION

Once the syringe has been assembled as described before, the same operates as a conventional syringe, the piston (123) being able to move with its stem (124) within the barrel (120), with the sole limitation that the piston (123) in not able to go far from the obstructions (126) adjacent to the flange (122). That is possible because within the movement of the piston (123) for the expulsion of liquid contained in the barrel (120), the cylindrical projections (144) lean against the free surface of the disk (125) and through the supports (143) they transmit movement to the guided set (150), which is completed with the the thrust of the body (127) and thrust projection (145) in the disk (125), against the free surface of the disk (138) between the projecting bodies (139), the free surfaces of which lean against the empty surface of the disk (125).

In the movement of the piston (123) for the suction of liquid through the adapter (121) without the application of a needle, the syringe allows engagement of the guiding (149) set with the guided (150) set to be maintained by virtue of the cylindrical projections (144)retained between the end of the wing (135) and the inner wall (132) of the body (127), as the empty space (134) is smaller than the diameter or the mentioned cylindrical projections (144).

When the needle is applied to the adapter (121), the suction flow is obviously considerably reduced, therefore a depression is caused between the adapter (121) and the stopper (137) of the guided set (150), said depression displacing the piston (123) in the suction movement of the liquid. This depression as it has been caused, tends to retain the guided set (150) against the displacement of the guiding set (149) so that the cylindrical projections (144) are forced through the space (134), the end of the wing (135) becoming loose and leaving the cylindrical projections against the projection (128) as is illustrated in figure 18.

Once the syringe has been filled with the liquid to be injected and once the needle has been stuck into the patient's body, the movement needed for the injection of the liquid between the stopper (137) of the guided set (150) and the adapter (121) is started; this causes a pressure area, as pressure is exercised in the guiding set (149) through the piston (123) as the outlet of the liquid is limited by the reduction of flow which the use of the needle implies, and there is resistance to the flow of liquid into the patient's body. Said pressure area makes the cylindrical projections (144), that were in the inner cavity which forms the projection (128), displace up to the surface (135) of the wing (133) and overcome the elastic resistance of the projection (128) so that they pass through the free space (136) as illustrated in figure 19, producing a disengagement of the guiding set (149) with respect to the guided set (150) as illustrated in figure 20. The retention exercised by the obstructions (126) obstructs the withdrawal of the plunger (124), so that the syringe may not be disassembled, becoming disabled for reuse. It is important to notice that the disengagement of the guiding (149) and guided (150) sets in produced without the voluntary intervention of the person applying the injection, as said disengagement is produced with the natural movements of suction and injection of the liquid in the syringe.

It is obvious that once this invention is taken into practice, modifications as regards certain construction and form details may be introduced, without this implying that the main principles clearly sustained in the following claims may be left aside.

## Claims

1. A hypodermic non-reusable syringe having a predetermined limited nimber of strokes and comprising a barrel which has a rear end opening and at the opposite end is closed by a bottom provided with an outlet which extends through an external tubular adapter in which a tubular needle is engageable as a channelling means, for liquid contained in or to be contained in the barrel; a piston slidable in and longitudinally of the barrel and connected to a controlling plunger the opposite end of which projects through the barrel rear end opening; the piston being connected to the plunger via an engaging and disengaging mechamism of the piston and plunger in their aspiration and injection action; and retaining means for the plunger being provided within the barrel of the syringe in a region where it does not interfere with the normal operation of the piston and plunger.

2. A syringe according to claim 1 having guiding means for said plunger and guided means for said piston, said guiding means forming a passage which has a first retaining portion and a second releasing portion for said guided means.

3. A syringe according to claim 2 in which said guided means comprises a support in the passage of the guiding means connected to said piston in a laterally displacable way with respect to the movement of the piston.

4. A syringe according to claim 3 wherein said support comprises two bolts one opposite the other and disposed in said passage of the guiding means, each joined by its external end to a respective leg elastically articulated to an upper part of the piston.

5. A syringe according to claim 4 wherein the piston has a circular base which has a retaining means of the cylindrical body forming the piston in its surface opposite the fixing of the legs, which makes contact in an hermetically displacing way with the inner part of the cylinder.

6. A syringe according to claims 4 and 5 wherein the circular base, between the legs and the inner wall of the barrel, has bodies each of external circular outline and with a flat inner wall parallel to said legs, said bodies being elastically articulated to said upper part of the piston.

7. A method for assembling a syringe according to any preceding claim which comprises placing the piston into the rear end opening of the barrel; arranging the guiding means on the central area of the base in such a way that the portions of the bolt projecting from the legs come opposite to the slides formed by said guiding means; thrusting the piston into the barrel to cause insertion of the portions of the bolt within the slides formed by the guiding means; and then folding wings which delimit the rear end opening of the barrel around the cross-section of the plunger.

8. A method according to claim 7 wherein the piston is introduced into the barrel to the limit, until said piston reaches the bottom.

9. A method according to claim 7 or 8 wherein the folding of the wings to delimit the rear end opening of the barrel is done under the action of heat.

10. A non-reusable syringe which comprises a barrel which has an open end while the opposite end is closed with a bottom provided with a pipe through an external tubular adapter for engaging a needle; a piston slidable in said barrel and directed by a plunger the opposite end of which projects from the open end of the barrel, the piston being connected to the plunger through an engaging and disengaging mechanism of the piston with respect to the plunger; and retaining means for the plunger disposed within the barrel of the syringe in a region where it does not interfere with the normal operation of the piston and the plunger; the engaging and disengaging mechanism comprising guiding means for said plunger and guided means for said piston with said guiding means being provided with a passage which has a first retaining portion and a second releasing portion for said guided means and said guided means having a projection disposed in said passage of the guiding means; and there being disposed in said passage elastic retaining means for said projection which allow movement of the engaged piston and plunger according to a determined condition of said retaining means and release of the projection according to another determined condition of said retaining means, for displacement of said projection in the retaining and releasing portions of said passage of the guiding means.

11. A syringe according to claim 10 wherein said elastic retaining means are constituted by a tab which is fixed to a base of the plunger and is opposite to the inclined plane of a body joined to said base, the separation between the end of said tab and the surface of said inclined plane being of smaller dimension than that of the projection of the guided means in the passage of the guiding means.

12. A syringe according to claim 11 wherein the passage is formed by the inclined wall of the body joined to the plunger, which has an end formed by a projection directed towards the plunger and separated from the upper surface of said tab by an amount slightly smaller than the dimension of the projection of the guided means in said passage so formed.
